# EUROPEAN PATENT APPLICATION

(11) **EP 0 648 843 A1**
(43) Date of publication of application: **19.04.1995**
(21) Application number: 94306856.9
(22) Date of filing: 20.09.1994
(51) Int. Cl.: C12N 15/56, C12N 9/28

(54) **DNA encoding a hyperthermostable alpha-amylase**

(30) Priority: 01.10.1993 JP 267777/93
(71) Applicant: TAKARA SHUZO CO. LTD., Fushimi-ku Kyoto 612 (JP)
(72) Inventor: Taguchi, Yuki, College Park, Maryland 20740 (US); Nishikawa, Mio, Shiga-ken (JP); Koyama, Nobuto, Shiga-ken (JP); Yamamoto, Katsuhiko, Shiga-ken (JP); Asada, Kiyozo, Shiga-ken (JP); Kato, Ikunoshin, Uji-shi, Kyoto-fu (JP)
(74) Representative: Wakerley, Helen Rachael

(57) **Abstract**

[Object] To provide a genetic engineering process for producing α-amylase with the use of an isolated hyperthermostable α-amylase gene.
[Constitution] A hyperthermostable α-amylase gene comprising a nucleotide sequence represented by SEQ ID NO: 1 of the sequence listing (obtained from Pyrococcus furiosus and having a sequence length of 1305). A hyperthermostable α-amylase gene hybridizable to the above hyperthermostable α-amylase gene. A process for producing a hyperthermostable α-amylase by culturing a transformant into which a recombinant plasmid containing the above hyperthermostable α-amylase gene has been introduced. At pH 5.5, the optimum temperature of the enzyme activity is in the range of 70 to 100°C. The enzyme activity is entirely maintained even after heating at 80°C for 6 hours, and at least 70% of the enzyme activity is maintained even after the heating at 95°C for 6 hours.

## Description

### [Field of Industrial Application]

This invention relates to a DNA encoding a hyperthermostable α-amylase useful as an industrial enzyme.

### [Prior Art]

The α-amylase is being utilized in a variety of important industrial processes not only in the food industry but also in other various industries, such as the alcohol producing and textile industries. A typical use of the α-amylase is found in a liquefaction of starch to be conducted in a glucose producing process for obtaining glucose, which is either processed into a sweetener and a nutriment or utilized as a starting material for isomerized sugar. The liquefied starch is also an important starting material for producing cyclodextrin and coupling sugar. Because of low solubility in water at medium temperature, starch is hardly hydrolyzed with α-amylase. Consequently, liquefaction of starch with an α-amylase is generally performed by first gelatinizing the starch at a temperature as high as about 100°C and then contacting the resultant starch with the α-amylase for liquefaction.

The pH value of the reaction mixture during the liquefaction is adjusted to an acidic region in order to suppress the formation of by-products. Especially, when the liquefaction is continuously followed by a saccharifying step, it is desired that the liquefaction be carried out in an acidic condition suitable for having glucoamylase employed in the saccharification fully exert its activities. Therefore, it is required that the α-amylase for use in the liquefaction of starch have high activity even at extremely high temperatures and in acidic regions.

The liquefaction of starch is performed by the use of, for example, α-amylase derived from Bacillus licheniformis (trade name: Termamyl, produced by Novo Nordisk) and α-amylase derived from Bacillus subtilis (trade name: Speedase, produced by The Nagase & Co., Ltd.). Although these are mesophile-derived enzymes, their thermostability is relatively high.

In this connection, it is anticipated that the hyperthermophilic bacteria adapted to high-temperature environment might have produced α-amylases having increased thermostabilities and being suitable for use in the above liquefaction.

It is known that hyperthermophile Pyrococcus furiosus and Pyrococcus woesei produce α-amylases [Applied and Environmental Microbiology, 56, 1985-1991 (1990); Archives of Microbiology, 155 (6), 572-578 (1991); FEMS Microbiology Letters, 71 (1-2), 21-26 (1990); and WO 90/11352].

### [Problem to be Solved by the Invention]

The use of the above α-amylases derived from bacteria of the genus Pyrococcus in the liquefaction of starch is anticipated because of their high thermostabilities. In the above documents, however, not only was the amount of the produced enzyme very small but also the culturing of the microorganism at high temperatures was inevitable for obtaining the enzyme. Therefore, the processes disclosed in these documents involved problems from the viewpoint of the use as industrial production processes. Further, in the above documents, the α-amylases were not purified. Thus, they failed to provide purified enzymes.

In view of the above problems, the development of a genetic engineering process for producing the above α-amylase is desired in the art.

The present invention are to isolate a DNA encoding α-amylase derived from Pyrococcus furiosus and to provide a above-mentioned DNA, thereby solving the above problems.

### [Means for Solving the Problem]

In a aspect of the present invention, there is provided an isolated DNA selected from the group consisting of:
(a) a DNA encoding a hyperthermostable α-amylase and having a nucleotide sequence selected from the group consisting of the sequences represented by SEQ ID No: 1, SEQ ID No: 2 and SEQ ID No: 3 of the sequence listing;
(b) a DNA capable of hybridizing to the DNA of (a) under stringent conditions and which encodes hyperthermostable α-amylase.

The stringent condition is as follows:
(1) A nylon membrane on which DNA is fixed is incubated with probe in the solution containing to final concentration 6xSSC(1xSSC contains 8.67g of NaCl and 4.41g of sodium citrate in one liter of water), 1% SDS, 100 µg/ml of salmon sperm DNA, and 5xDenhardt's, which contains 0.1% bovine serum albumin, 0.1% polyvinyl-pirrolidone, and 0.1% Ficoll at 65°C for 20 hours.
(2) The nylon membrane is first washed with 2xSSC containing 0.1% SDS at 37°C and either washing temperature is increased every three degrees or SSC concentration is decreased every ten percents starting from the original condition until a signal derived from DNA on the membrane and that from background are distinguishable.

The inventors independently attempted to purify the α-amylase derived from Pyrococcus furiosus DSM 3638 and determine the amino acid sequence of a part of the enzyme with a view toward obtaining the DNA encoding the α-amylase. However, the purification of the enzyme was difficult, and the inventors failed to obtain an enzyme preparation having a purity that permit determination of the partial amino acid sequence.

The expression cloning method permits cloning of an gene of the enzyme in the absence of any information on the primary structure of the desired enzyme. For example, pullulanase gene was obtained from Pyrococcus woesei by this method (W0 92/02614). However, the method cannot be applied to cloning of any type of enzyme because in case the plasmid vector is used for the method, a very suitable restriction enzyme is needed; It must cleave the target DNA into small size enough to be inserted in a plasmid vector and neither cleave the target gene at an inner site. Furthermore, the method is complicated because it needs a number of clones.

Subsequently, the present inventors have attempted to isolate the DNA encoding the α-amylase by screening α-amylase activity in a cosmid library constructed with Pyrococcus furiosus genomic DNA and a cosmid vector, in which larger DNA fragments (35-50kb) can be inserted than in plasmid vectors. By using cosmid vectors, possibilities for cleaving an inner site of the target DNA encoding the enzyme by a restriction enzyme decrease and the numbers of clones necessary to test can be reduced. On the contrary, the cosmid vectors have less copy numbers in host organisms than the plasmid vectors, so that it may be difficult to detect the enzyme activity which is expressed at low level.

To inventors surprise, they realized that some cosmid clones did express proteins that are encoded by Pyrococcus furiosus DNA and also noticed that high thermostability of the target enzyme could be of great advantage for screening of its gene. Lysates of thermostable proteins was quite efficiently prepared being separated from thermolabile ones derived from host cells. The inventors cultivated each of the transformants in the cosmid library and prepared lysates containing only the thermostable proteins. The group of these lysates is named as "cosmid protein library". By using the library thus constructed for detection of the enzyme activity, detection sensitivity increases than using colonies on a petri dish of the transformants and disadvantages such as background or inhibition of enzyme activity caused by proteins from hosts can be removed.

The present inventors isolated several cosmid clones which show α-amylase activity by screening the cosmid protein library constructed with Pyrococcus furiosus genomic DNA. Furthermore, the present inventors isolated the gene coding a hyperthermostable α-amylase from the DNA fragments inserted in the clones isolated above by making full use of various genetic engineering techniques, and determined the DNA sequence of the gene. And more, the present inventors succeeded in the expression of the hyperthermostable α-amylase with the use of the gene, thus completing the present invention.

The expression cloning method using cosmid vectors described here cannot be always applied to any thermostable enzyme. The result is determined by the property of the target gene. For the example, the present inventors attempted to isolate the DNA encoding a protease of Pyrococcus furiosus (Applied and Environmental Microbiology, 56, 1992-1998 (1990)), but they didn't reach to the isolation of the gene.

It is known that most of the α-amylases having undergone gene analysis are first synthesized as precursor proteins and, subsequently, converted to mature enzymes with the signal peptide cut off [Applied Microbiology and Biotechnology, 23, 355-360 (1986)].

The inventors have succeeded in producing the desired α-amylase by determining the nucleotide sequence of an isolated DNA encoding hyperthermostable α-amylase, comparing it with that of the known DNA encoding α-amylase to thereby presume a region coding for the mature α-amylase, constructing a recombinant plasmid capable of expressing the mature α-amylase in a microorganism, and cultivating the microorganism transformed with the recombinant plasmid, and thus have completed the present invention.

The DNA encoding the hyperthermostable α-amylase according to the present invention can be obtained by screening a gene library of hyperthermophiles. The hyperthermophile is, for example, a bacterium belonging to the genus Pyrococcus. Thus, for example, the desired gene can be obtained by screening a cosmid library of the genome of Pyrococcus furiosus. For this purpose, Pyrococcus furiosus DSM3638 can be employed, which is available from Deutsche Sammlung von Mikroorganismen und Zellkulturen GmbH.

The cosmid library of the genome of Pyrococcus furiosus can be obtained by partially digesting the genomic DNA of Pyrococcus furiosus with a restriction enzyme Sau3A I (produced by Takara Shuzo Co., Ltd.), ligating the resultant DNA fragments to triple helix cosmid vector (produced by Stratagene), packaging in lambda phage particles by the in vitro packaging method, and transforming an appropriate strain of Escherichia coli, e.g., Escherichia coli DH5αMCR (produced by BRL). A group of lysates containing thermostable proteins expressed in Escherichia coli, which is named cosmid protein library, can be prepared as follows: each of bacterial cells obtained by cultivating the transformants is heated at 100°C for 10 minutes, sonicated, and heated at 100°C for 10 minutes again, centrifuged, and supernatant is collected. The α-amylase activity in this cosmid protein library is examined using starch as substrate. In this manner, some cosmid clones each containing a DNA encoding a hyperthermostable α-amylase can be obtained which express α-amylases resistant to the above heat treatment.

Further, a cosmid DNA prepared from the thus obtained cosmid clone containing the DNA encoding the hyperthermostable α-amylase may be digested to some DNA fragments with appropriate restriction enzymes for construction of recombinant plasmids containing the above DNA fragments. The recombinant plasmids are introduced to an appropriate microorganism to thereby obtain transformants. The activity of the α-amylase produced by each of the transformants is examined and the recombinant plasmid containing the desired DNA encoding the hyperthermostable α-amylase can be obtained.

More specifically, the cosmid DNA prepared from one of the above cosmid clones is digested with Hind III (produced by Takara Shuzo Co., Ltd.), and the resultant DNA fragments are inserted in plasmid vector pUC119 (produced by Takara Shuzo Co., Ltd.) at its Hind III site. Subsequently, the recombinant plasmids are introduced to Escherichia coli JM109 (produced by Takara Shuzo Co., Ltd.), and the resultant transformants are grown on a nitrocellulose membrane. Cells on the membrane are lysed with chloroform vapor, and the membrane is put on an agarose gel containing starch and incubated. After the incubation, starch degradation is examined by the iodine-starch reaction to thereby detect the α-amylase activity of each of the transformants. Thus, a recombinant plasmid containing a DNA encoding α-amylase which express a hyperthermostable α-amylase activity can be obtained.

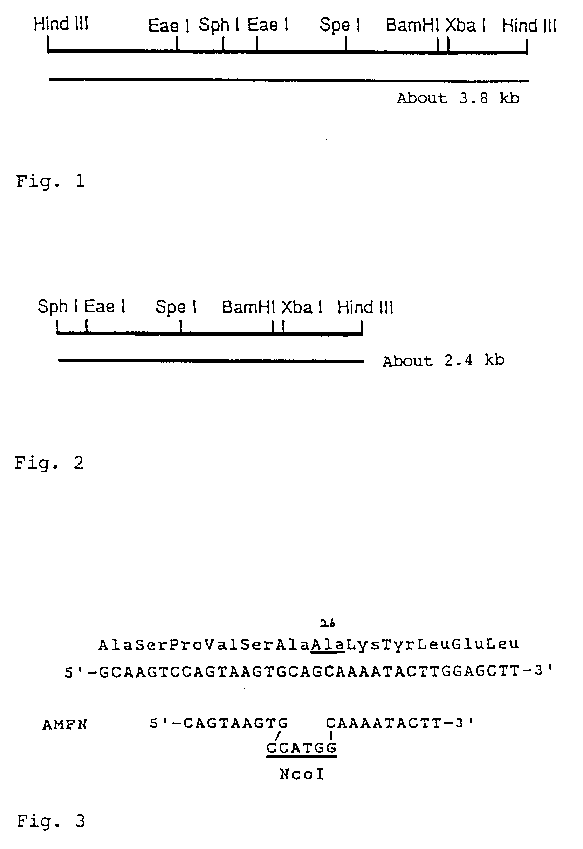

The obtained plasmid was designated plasmid pHI86, and the Escherichia coli JM109 transformed with the plasmid was designated as Escherichia coli JM109/H-86. This strain was deposited on July 16, 1993 at National Institute of Bioscience and Human-Technology Agency of Industrial Science and Technology (1-3, Higashi 1 chome Tsukuba-shi Ibaraki-ken 305, JAPAN), under the accession number FERM P-13759, and on July 29, 1994 this deposit was converted to deposit at National Institute of Bioscience and Human-Technology Agency of Industrial Science and Technology in accordance with the Budapest Treaty under the accession number FERM BP-4763. Fig. 1 shows the restriction map of the DNA fragment inserted in the plasmid pHI86.

The plasmid pHI86 is digested with various restriction enzymes to produce DNA fragments, and the DNA fragments are inserted in an appropriate plasmid vector to thereby obtain recombinant plasmids. Escherichia coli JM109 is transformed with the recombinant plasmids, and the α-amylase activity of each of the transformants is examined. Thus, the DNA fragments which do not contain DNA encoding hyperthermostable α-amylase can be deleted from the plasmid pHI85. Specifically, the above plasmid pHI86 is digested with Hind III and Sph I (produced by Takara Shuzo Co., Ltd.), and DNA fragment of about 2.4 kb is isolated and ligated to Hind III and Sph I sites of plasmid vector pTV118N (produced by Takara Shuzo Co., Ltd.). The obtained recombinant plasmid is introduced to Escherichia coli JM109. The α-amylase activity of each of the resultant colonies is examined by the above method employed in the screening of the cosmid protein library, and the plasmid is prepared from the colony expressing the α-amylase activity. The obtained recombinant plasmid was designated plasmid pSH24, and the Escherichia coli JM109 transformed with the plasmid was designated Escherichia coli JM109/pSH24. Fig. 2 shows the restriction map of the DNA fragment inserted in the plasmid pSH24.

The nucleotide sequence of the above plasmid pSH24 can be determined by any of the conventional methods, e.g., the dideoxy method.

Specifically, various deletion mutants of the plasmid pSH24 having part of the inserted DNA fragment are prepared, and the nucleotide sequence are determined by the dideoxy method using suitable ones among the deletion mutants as templates. Further, the nucleotide sequence of the DNA fragment containing the DNA encoding the hyperthermostable α-amylase which has been inserted in the plasmid pSH24 can be determined by comparing and analyzing the each of the nucleotide sequences of the deletion mutants.

The nucleotide sequence of the Eae I-Hind III region of the DNA fragment inserted in the plasmid pSH24 is shown in SEQ ID NO: 3 of the sequence listing. That is, the SEQ ID NO : 3 of the sequence listing is an example of the nucleotide sequence of the DNA fragment containing the DNA encoding the hyperthermostable α-amylase obtained according to the present invention. The amino acid sequence of the hyperthermostable α-amylase deduced from the above nucleotide sequence is shown in SEQ ID NO: 4 of the sequence listing.

A recombinant plasmid capable of expressing a hyperthermostable mature α-amylase Escherichia coli can be constructed on the basis of the above amino acid sequence. That is, the position of the N-terminal amino acid of the mature α-amylase can be presumed by comparing the above amino acid sequence with various α-amylases whose amino acid sequences are known [Applied Microbiology and Biotechnology, 23, 355-360 (1986)].

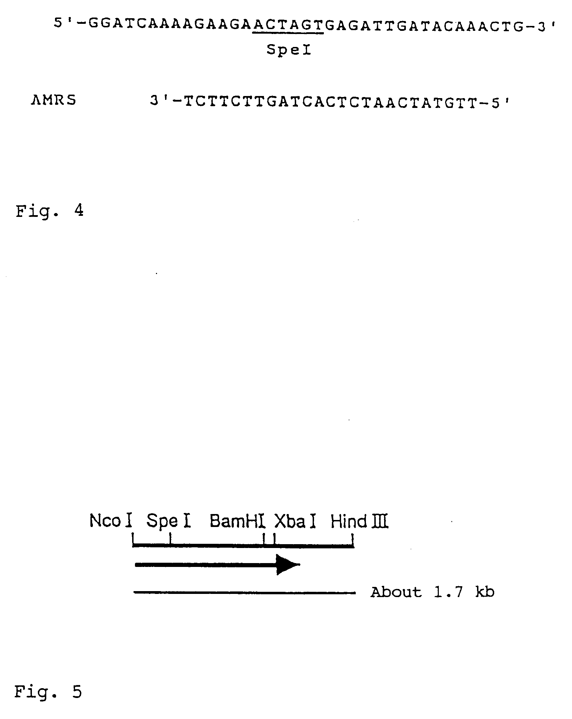

Oligonucleotide AMFN (shown in Fig. 3) having a nucleotide sequence shown in SEQ ID NO: 5 of the sequence listing, which can introduce the initiation codon (ATG) and the Nco I site (CCATGG) immediately ahead of the codon for the above N-terminal amino acid of the mature α-amylase on the plasmid pSH24, and oligonucleotide AMRS (shown in Fig. 4) having a nucleotide sequence shown in SEQ ID NO: 6 of the sequence listing, which is complementary to a region including the Spe I site of the plasmid pSH24, are synthesized. PCR is performed with the use of the above oligonucleotides as primers and the plasmid pSH24 as a template, thereby amplifying a DNA fragment having the above two oligonucleotides at each terminals and including part of the DNA encoding the hyperthermostable α-amylase. This DNA fragment is digested with Nco I (produced by Takara Shuzo Co., Ltd.) and Spe I (produced by Takara Shuzo Co., Ltd.), ligated to about 4.6 kb Nco I-Spe I fragment of the plasmid pSH24, and introduced to Escherichia coli JM109. The resultant colonies are cultivated in the presence of IPTG, and the α-amylase activity of each of the colonies is examined in accordance with the above method employed in the screening of cosmid protein library. The plasmid is prepared from a colony expressing the α-amylase activity.

The obtained plasmid was designated plasmid pNH17, and the Escherichia coli JM109 transformed with the plasmid was designated Escherichia coli JM109/pNH17. This strain was deposited on September 10, 1993 at National Institute of Bioscience and Human-Technology Agency of Industrial Science and Technology (1-3, Higashi 1 chome Tsukuba-shi Ibaraki-ken 305, JAPAN), under the accession number FERM P-13859, and on July 29, 1994 This deposit was converted to deposit at National Institute of Bioscience and Human-Technology Agency of Industrial Science and Technology in accordance with the Budapest Treaty under the accession number FERM BP-4764.

Fig. 5 shows the restriction map of the DNA fragment inserted in the plasmid pNH17. In the figure, the bold arrow indicates the region coding for the hyperthermostable α-amylase protein. The nucleotide sequence of the DNA fragment inserted in the plasmid pNH17 is shown in SEQ ID NO: 2 of the sequence listing. That is, the SEQ ID NO: 2 of the sequence listing is an example of the nucleotide sequence of the DNA fragment containing the DNA encoding the hyperthermostable α -amylase obtained according to the present invention.

Furthermore, the nucleotide sequence of the DNA fragment encoding the hyperthermostable α-amylase, which is illustrated with bold arrow in Fig. 5, is shown in SEQ ID NO: 1 of the sequence listing. That is, the SEQ ID NO: 1 of the sequence listing is the hyperthermostable α-amylase gene obtained according to the present invention. The amino acid sequence of the mature hyperthermostable α-amylase deduced from above nucleotide sequence is shown in SEQ ID NO: 7 of the sequence listing.

When Escherichia coli JM109/pNH17 is cultivated under conventional conditions, e.g., at 37°C in LB medium (1% trypton, 0.5% yeast extract, and 0.5% NaCl; pH 7.0) containing 50 µg/ml ampicillin and when IPTG is added at an appropriate time during the cultivation, the bacterial cells express the hyperthermostable α -amylase. The bacterial cells are harvested from culture, sonicated, and centrifuged to thereby obtain a supernatant as a cell-free extract. The cell-free extract is heated at 95°C for 30 minutes to denature the thermolabile proteins, and nucleic acid removal, salting-out with ammonium sulfate and dialysis are carried out to thereby obtain a partially purified hyperthermostable α-amylase preparation.

The enzymatic and physicochemical properties of the partially purified hyperthermostable α-amylase produced by Escherichia coli JM109/pNH17 are as follows.

### (1) Function

The α-amylase is capable of hydrolyzing starch and thus reducing the coloration exhibited by the iodine-starch reaction. Further, it hydrolyzes and liquefies gelatinized starch.

### (2) Assay for enzyme Activity

Enzyme activity was measured by spectrophotometrically following the reduction of coloration due to the iodine-starch reaction conducted after the enzymatic hydrolysis of soluble starch as a substrate. Specifically, the enzyme preparation to be assayed was appropriately diluted, and 50 µl of the resultant preparation was added to 200 µl of 50 mM sodium phosphate buffer (pH 5.5) containing 0.5% soluble starch (produced by Merck & Co., Ltd.), and incubated at 95°C for 15 minutes.

The reaction mixture was cooled on ice to thereby stop the reaction, and 500 µl of a 0.01 N iodine solution (containing 1% potassium iodide) was added thereto and mixed. An aliquot of 50 µl was withdrawn from the mixture, diluted 10-fold with distilled water, and the absorbance at 600 nm was measured.

One unit of the enzyme was defined as the amount of the enzyme with the absorbance at 600 nm was reduced at 95°C by 1% per minute. The hyperthermostable α -amylase preparation obtained according to the present invention exhibited a starch hydrolyzing activity when measured at 95°C and at pH 5.5.

Further, the liquefaction activity of the enzyme was measured with the use of corn starch as a substrate. Specifically, 1.5 ml of 50 mM sodium phosphate buffer containing 50 units of the enzyme was added to 1.5 ml of 50 mM sodium phosphate buffer (pH 5.5) containing 20% corn starch (produced by Nacalai Tesque). The mixture was gelatinized with agitating on a boiling water bath, then incubated at 95°C for 60 minutes. The viscosity of the reaction mixture markedly decreased as compared with that of a control in which the enzyme was not added. The hyperthermostable α -amylase preparation obtained according to the present invention exhibited starch liquefaction activity when measured at 95°C and at pH 5.5.

The enzymatic activity mentioned in the following items (3) to (5) was one determined by the above activity assay method utilizing the iodine-starch reaction.

### (3) Optimum pH

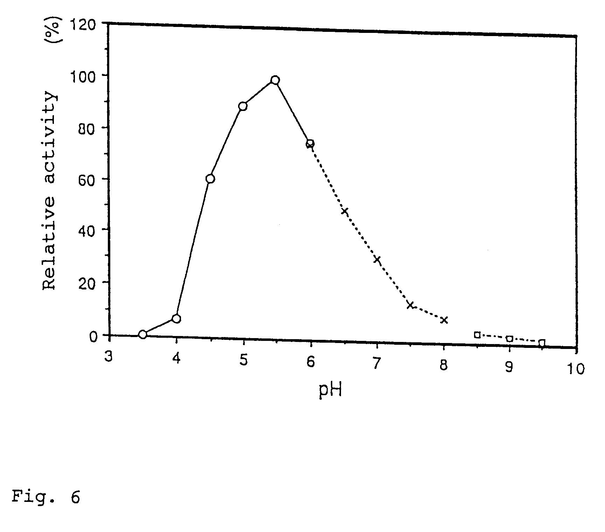

In order to investigate the optimum pH, the enzyme activity was measured with substrate solution adjusted to various pH. The substrate solutions were prepared with the following buffers: 50 mM sodium acetate buffer for pH 3.5 to 6.0, 50 mM potassium phosphate buffer for pH 6.0 to 8.0, and 50 mM sodium borate buffer for pH 8.5 to 9.5. Fig. 6 shows the relationship between the pH and the activity of the hyperthermostable α-amylase preparation obtained according to the present invention. The ordinate indicates the relative activity (%) of the enzyme while the abscissa indicates the pH of the reaction mixture in the figure. Further, in the figure, the open circle, the cross, and the open square, respectively, indicate the results obtained with the use of the 50 mM sodium acetate buffer, the 50 mM potassium phosphate buffer, and the 50 mM sodium borate buffer. It is apparent from Fig. 6 that the hyperthermostable α-amylase preparation of the present invention is active at pH values ranging from 4.5 to 6.5 and exhibits the highest activity at pH 5.5.

### (4) Optimum Temperature

The enzyme activities were measured at various temperatures to investigate the optimum temperature.

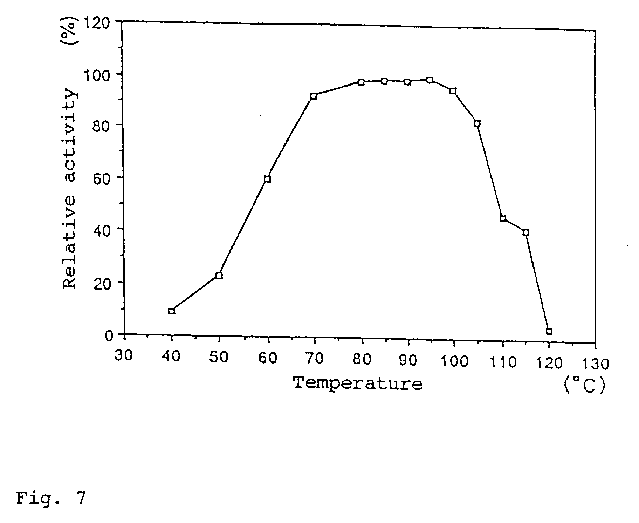

Fig. 7 shows the relationship between the temperature and the activity of the hyperthermostable α-amylase preparation obtained according to the present invention. The ordinate indicates the relative activity (%) of the enzyme while the abscissa indicates the temperature (°C) of the reaction mixture in the figure. It is apparent from Fig. 7 that the hyperthermostable α-amylase preparation of the present invention is highly active at temperatures ranging as broadly as 70 to 100°C when measured at a pH value of 5.5.

### (5) Thermostability

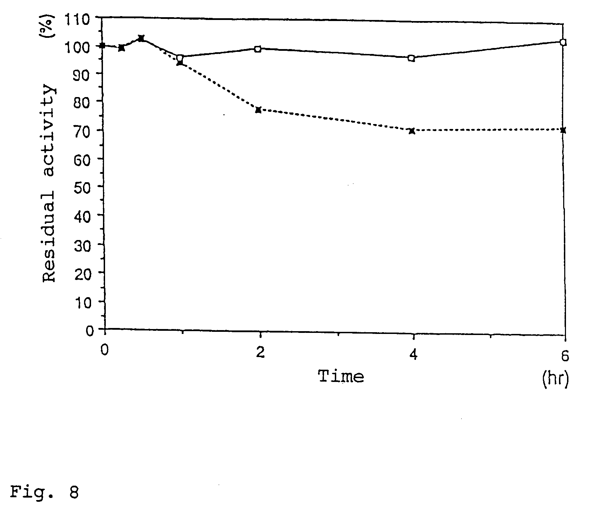

The enzyme solution was incubated at each of 80 and 95°C for various periods, and the change of residual enzyme activity was measured to investigate the thermostability of the enzyme. Fig. 8 shows the thermostability of the hyperthermostable α-amylase preparation obtained according to the present invention. The ordinate indicates the residual activity (%) of the enzyme while the abscissa indicates the time (hr) during which the enzyme solution was incubated in the figure. It is apparent from Fig. 8 that the activity of the hyperthermostable α-amylase preparation of the present invention does not decrease even when the enzyme solution is incubated at 80°C for 6 hours and at least 70% of the initial activity still remains even when the enzyme solution is incubated at 95°C for 6 hours.

As described in detail above, the present invention provides the DNA encoding the hyperthermostable α-amylase. Further, the present invention can produce the industrial process of the hyperthermostable α-amylase with the use of the above DNA.

The α-amylase encoded by the gene of the present invention can be used in a variety of important industrial processes not only in the food industry but also in other various industries, such as the alcohol producing and textile industries. A typical use of the α-amylase encoded by the gene of the present invention is found in a liquefaction of starch to be conducted in a process for obtaining glucose, which can be either processed into a sweetener and a nutriment or utilized as a starting material for an isomerized sugar. The liquefied starch is also an important starting material for producing cyclodextrin and coupling sugar. Liquefaction of starch with an α-amylase encoded by the gene of the present invention can be performed by contacting starch directly with the α-amylase immediately after gelatinizing the starch at a temperature as high as about 100°C.

The pH value of the reaction mixture during the liquefaction is adjusted to an acidic region in order to suppress the formation of by products. Especially, when the liquefaction is continuously followed by a saccharifying step, it is desired that the liquefaction be carried out in an acidic condition suitable for having glucoamylase employed in the saccharification fully exert its activities. Because the α-amylase encoded by the gene of the present invention have high activity even at extremely high temperatures and in acidic regions, it is quite useful for liquefaction of starch.

In addition, all of the gene which is similar, but not same to the gene obtained according to the present invention and which is expected to express the same enzyme activity may be obtained by hybridization in stringent condition using the gene, the DNA fragment containing the gene encoding the hyperthermostable α -amylase obtained according to the present invention, or appropriate part of the nucleotide sequence of the gene as probe.

Moreover, all of the gene which is similar, but not same to the gene obtained according to the present invention and expected to express the same enzyme activity may be obtained by PCR using appropriate part of the nucleotide sequence of the gene encoding the hyperthermostable α-amylase obtained according to the present invention as primers.

Furthermore, the DNA encoding the hyperthermostable α-amylase obtained according to the present invention may be appropriately modified so as to further improve the functions of the α-amylase.

### [Brief Description of the Drawings]

### [Fig. 1]

A figure showing the restriction map of the DNA fragment of about 3.8 kb containing the DNA encoding the hyperthermostable α-amylase.

### [Fig. 2]

A figure showing the restriction map of the DNA fragment of about 2.4 kb containing the DNA encoding the hyperthermostable α-amylase.

### [Fig. 3]

A figure showing the sequence of oligonucleotide AMFN.

### [Fig- 4]

A figure showing the sequence of oligonucleotide AMRS.

### [Fig. 5]

A figure showing the restriction map of the DNA fragment of about 1.7 kb containing the DNA encoding the hyperthermostable α-amylase.

### [Fig. 6]

A figure showing the relationship between the pH and the activity of the hyperthermostable α-amylase obtained according to the present invention.

### [Fig. 7]

A figure showing the relationship between the temperature and the activity of the hyperthermostable α-amylase obtained according to the present invention.

### [Fig. 8]

A figure showing the thermostability of the hyperthermostable α-amylase obtained according to the present invention.

### [Examples]

Embodiments of the present invention will now be set out, which in no way limit the scope of the invention.

In the Examples, % means % by weight.

### Example 1

### (Preparation of Pyrococcus furiosus genome DNA)

Pyrococcus furiosus DSM 3638 was incubated in the following manner.

Two l of a medium comprising 1% of trypton, 0.5% of yeast extract, 1% of soluble starch, 3.5% of Jamarin S Solid (produced by Jamarin Laboratory), 0.5% of Jamarin S Liquid (produced by Jamarin Laboratory), 0.003% of MgSO₄, 0.001% of NaCl, 0.0001% of FeSO₄ 7H₂O, 0.0001% of CoSO₄, 0.0001% of CaCl₂ 7H₂O, 0.0001% of ZnSO₄, 0.1 ppm of CuSO₄ 5H₂0, 0.1 ppm of KAl(SO₄)₂, 0.1 ppm of H₃BO₃, 0.1 ppm of Na₂MoO₄ 2H₂O, and 0.25 ppm of NiCl₂ · 6H₂O was fed into a 2 l medium bottle and sterilized at 120°C for 20 minutes. After eliminating the dissolved oxygen by blowing nitrogen gas, the medium was inoculated with the above-mentioned strain, which was then stationarily incubated at 95°C for 16 hours. After the completion of the incubation, cells were collected by centrifugation.

Then the collected cells were suspended in 4 ml of a 0.05 M Tris-HCl (pH 8.0) containing 25% of sucrose. To the obtained suspension were added 0.8 ml of lysozyme [5 mg/ml, 0.25 M Tris-HCl (pH 8.0)] and 2 ml of 0.2 M EDTA. After maintaining at 20°C for 1 hour, 24 ml of an SET solution [150 mM NaCl, 1 mM EDTA, 20 mM Tris-HCl (pH 8.0)] was added. Further, 4 ml of 5% SDS and 400 µl of proteinase K (10 mg/ml) were added thereto, followed by a reaction at 37°C for 1 hour. After the completion of the reaction, the reaction mixture was extracted with chloroform/phenol and precipitated with ethanol. Thus approximately 3.2 mg of a genome DNA was prepared.

### (Preparation of cosmid protein library)

400 µg of the Pyrococcus furiosus DSM 3638 genomic DNA was partially digested with Sau3A I and fractionated according to the size by density gradient centrifugation. One µg of Triple Helix Cosmid Vector was cleaved with BamH I and mixed with 140 µg of the genomic DNA fragments of 35 to 50 kb which had been obtained by the fractionation as described above. After ligating with the use of a Ligation Kit (produced by Takara Shuzo Co., Ltd.), the Pyrococcus furiosus genomic DNA fragments were packaged into lambda phage particles by the in vitro packaging method using Gigapack II Gold (produced by Stratagene). By using a part of the phage solution thus obtained, Escherichia coli DH5αMCR was transformed to thereby give a cosmid library.

First cosmid DNA was prepared from several colonies thus obtained and it was confirmed that they had an inserted fragment of an appropriate size in common. Next, each of 500 colonies was suspended in 150 ml of LB medium containing 100 µg/ml of ampicillin and incubated under shaking at 37°C for 16 hours. The culture was centrifuged and cells were collected as precipitate. These cells were suspended in 20 mM Tris-HCl (pH 8.0) and treated at 100°C for 10 minutes. Subsequently, they were sonicated and further treated at 100°C for 10 minutes.

The lysate obtained as supernatant after centrifugation was used as a cosmid protein library.

### (Selection of cosmid clones containing DNA encoding hyperthermostable α-amylase)

The α-amylase activity was detected by spectro-photometrically following the reduction of coloration due to the iodine-starch reaction which occurred by the hydrolysis of starch. Specifically, 20 µl of lysate were withdrawn from the above cosmid protein library, and 50 µl of 0.25 M sodium phosphate buffer containing 0.04% of soluble starch (pH 5.5) was added thereto. The mixture was incubated at 95°C for 30 minutes, and 50 µ l of 0.01 N iodine solution (containing 1% potassium iodide) and 150 µl of distilled water were added thereto. Thereafter, the absorbance at 630 nm was measured and compared with that of a control. Thus, nine cosmid clones having α-amylase activities were obtained from the cosmid protein library.

### (Construction of plasmid pHI86)

A cosmid DNA was prepared from one of the nine cosmid clones which exhibited the highest α-amylase activity, digested with each of BamH I, EcoR I, Hind III, and Pst I, and ligated to the multicloning site of a plasmid vector pUC119. The resultant recombinant plasmid was introduced to Escherichia coli JM109 and spread on an LB plate containing 50 µg/ml ampicillin. The α-amylase activity of each of the thus formed colonies was measured. Namely, the formed colonies were transferred onto a nitrocellulose membrane, and incubated on an LB plate containing 50 µg/ml ampicillin at 37°C overnight. Subsequently, the membrane was incubated in chloroform vapor for 15 minutes to thereby cause the colonies to undergo bacteriolysis. The resultant membrane was dried in air and placed, with its colony side down, on an agarose plate containing 1% soluble starch and a 50 mM sodium phosphate buffer (pH 5.5). After the incubation at 70°C overnight, the membrane was removed and a 0.01 N iodine solution (containing 1% potassium iodide) was applied to the plate to thereby color the starch. Colonies having α-amylase activities, which had no coloration with the iodine solution occurred because of the hydrolysis of starch were selected.

Colonies exhibiting the α-amylase activity were found among the colonies of the transformants prepared with the use of the three restriction enzymes exclusive of BamH I. Plasmid was prepared from each of the colonies. The size of the DNA fragment inserted in each of the resultant plasmids was determined. The recombinant plasmids constructed with EcoR I, Hind III, and Pst I contained DNA fragments of about 6.5, about 3.8, and about 8 kb, respectively. The recombinant plasmid containing the smallest DNA fragment which was constructed with Hind III was designated pHI86. The Escherichia coli JM109 transformed with the above plasmid was designated Escherichia coli JM109/H-86. This strain was deposited on July 16, 1993 at National Institute of Bioscience and Human-Technology Agency of Industrial Science and Technology (1-3, Higashi 1 chome Tsukuba-shi Ibaraki-ken 305, JAPAN), under the accession number FERM P-13759, and on July 29, 1994. This deposit was converted to deposit at National Institute of Bioscience and Human-Technology Agency of Industrial Science and Technology in accordance with the Budapest Treaty under the accession number FERM BP-4763. Fig. 1 shows the restriction map of the DNA fragment inserted in the plasmid pHI86.

### (Construction of plasmid pSH24)

The above plasmid pHI86 was digested with Hind III and Sph I, and a DNA fragment of about 2.4 kb was isolated with agarose gel electrophoresis. This DNA fragment was ligated to a plasmid vector pTV118N digested with Hind III and Sph I, and introduced to Escherichia coli JM109. The α-amylase activity of each of the formed colonies was examined by the method employed in the screening of the cosmid protein library.

A plasmid was prepared from the colony having exhibited α-amylase activities, and designated plasmid pSH24. The Escherichia coli JM109 transformed with the above plasmid pSH24 was designated Escherichia coli JM109/pSH24. Fig. 2 shows the restriction map of the DNA fragment inserted in the plasmid pSH24.

### Example 2

### (Determination of nucleotide sequence of DNA encoding the hyperthermostable α-amylase)

A series of deletion mutants were constructed from the plasmid pSH24 in order to determine the nucleotide sequence of the DNA fragment containing the DNA encoding the hyperthermostable α-amylase. A Kilo sequence deletion kit (produced by Takara Shuzo Co., Ltd.) was used in the construction of the deletion mutants. Some containing inserted DNA fragments with appropriate lengths were selected from among the deletion mutants, and the nucleotide sequence of each of the insert moieties was analyzed by the dideoxy method using the BcaBEST dideoxy sequencing kit (produced by Takara Shuzo Co., Ltd.). The data were combined and, consequently, the nucleotide sequence of the DNA fragment inserted in the plasmid pSH24 was determined. SEQ ID NO: 3 of the sequence listing shows nucleotide sequence from Eae I site to Hind III site with 2134 bp long as part of the nucleotide sequence of the DNA fragment containing the DNA encoding the hyperthermostable α-amylase, which was inserted in the plasmid pSH24.

Similarity exists between the nucleotide sequence from position 337 to 373 of the above sequence and the promoter sequence of archaebacteria [Nucleic Acids Research, 14, 2459-2479 (1986); and Nucleic Acids Research, 16, 1-19 (1988)], and SD-like sequence exists between position 380 and 388 of the above nucleotide sequence. Therefore, it is suggested that the hyperthermostable α-amylase is coded for with the GTG starting with the G at position 395 of the above nucleotide sequence as an initiation codon. On the other hand, the termination codon is TGA starting with the T at position 1775 of the above nucleotide sequence. The amino acid sequence of the hyperthermostable α-amylase deduced from the above nucleotide sequence is shown in SEQ ID NO: 4 of the sequence listing.

All of four highly homologous regions found in the known α-amylases [Applied Microbiology and Biotechnology, 23, 355-360 (1986)] were present in the above amino acid sequence. It is apparent from comparison of the N terminal region that 25 amino acid sequence, from Met 1 to Ala 25, of the above amino acid sequence correspond to a signal peptide, and that the N terminal reside of the mature α-amylase is the Ala 26.

### (Preparation of plasmid pNH17)

A recombinant plasmid capable of expressing a hyperthermostable mature α-amylase in Escherichia coli was constructed on the basis of the above obtained results by the site-directed mutagenesis utilizing PCR. First, oligonucleotide AMFN (SEQ ID NO: 5) so designed as to introduce the Nco I site and initiation codon immediately ahead of the codon for the N terminal amino acid of the mature α-amylase as shown in Fig. 3, and oligonucleotide AMRS (SEQ ID NO: 6) complementary to the region containing the Spe I site of the hyperthermostable α-amylase gene as shown in Fig. 4, were chemically synthesized. A DNA fragment of about 300 bp having the above oligonucleotide sequences at each ends was amplified by PCR in which the above two oligonucleotides were used as primers and the plasmid pSH24 was used as template. Thus amplified DNA fragment was digested with Nco I and Spe I. Separately, the plasmid pSH24 was digested with Nco I and Spe I, and a DNA fragment of about 4.6 kb was isolated therefrom by agarose gel electrophoresis. This DNA fragment was ligated to the above PCR product digested with restriction enzymes and introduced to Escherichia coli JM109.

The resultant colonies were cultivated in the presence of IPTG and the α-amylase activities thereof were examined by the method employed in the screening of the cosmid protein library. A plasmid was prepared from the colonies having exhibited activities and designated plasmid pNH17. The Escherichia coli JM109 transformed with the plasmid pNH17 was designated as Escherichia coli JM109/pNH17. This strain was deposited on September 10, 1993 at National Institute of Bioscience and Human-Technology Agency of Industrial Science and Technology (1-3, Higashi 1 chome Tsukuba-shi Ibaraki-ken 305, JAPAN), under the accession number FERM P-13859, and on July 29, 1994 this deposit was converted to deposit at National Institute of Bioscience and Human-Technology Agency of Industrial Science and Technology in accordance with the Budapest Treaty under the accession number FERM BP-4764. Fig. 5 shows the restriction map of the DNA fragment inserted in the plasmid pNH17. The nucleotide sequence of the DNA fragment inserted in the plasmid pNH17 is shown in SEQ ID NO: 2 of the sequence listing.

Furthermore, the coding region for the hyperthermostable α-amylase is illustrated with bold arrow in Fig. 5 and the nucleotide sequence of this part is shown in SEQ ID NO: 1 of the sequence listing. The amino acid sequence of the mature hyperthermostable α-amylase deduced from above sequence is shown in SEQ ID NO: 7 of the sequence listing.

### Example 3

### (Preparation of Partially purified hyperthermostable α-amylase)

The Escherichia coli JM109/pNH17 obtained in Example 2 was cultivated aerobically in 5 ml of an LB medium (1% trypton, 0.5% yeast extract, and 0.5% NaCl; pH 7.0) containing 50 µg/ml ampicillin at 37°C overnight. 500 ml of the above medium was prepared in a 2 l Erlenmeyer flask, and inoculated with 5 ml of the above culture. The mixture was cultivated aerobically at 37°C and when the absorbance at 660 nm of the culture reached 0.5, IPTG was added to a final concentration of 2 mM. The mixture was further cultivated at 37°C for 12 hours. Cells were harvested from culture by centrifugation and suspended in 5 ml of a 50 mM sodium phosphate buffer (pH 5.5). The suspension was sonicated to disrupt the cells and centrifuged to obtain a cell-free extract. The cell-free extract was heated at 95°C for 30 minutes and centrifuged to remove denatured protein. Streptomycin sulfate was added to the resultant supernatant to a final concentration of 1%.

The thus obtained solution was recentrifuged and ammonium sulfate was added to the resultant supernatant to 80% saturation. The formed precipitates were recovered by centrifugation, dissolved in 1 ml of a 50 mM sodium phosphate buffer (pH 5.5), and dialyzed against the above buffer overnight. Thus, partially purified enzyme preparation was obtained as the dialysate.

### [Effect of the Invention]

The gene coding for α-amylase having surprisingly high thermostability was obtained by virtue of the present invention. The hyperthermostable α-amylase of high purity can be supplied in large quantities by the use of the above gene.

Sequence Listing

SEQ ID NO:1

SEQUENCE LENGTH: 1305

SEQUENCE TYPE:nucleic acid

STRANDEDNESS:double

TOPOLOGY:linear

MOLECULE TYPE:Genomic DNA

HYPOTHETICAL:NO

ANTI-SENSE:NO

ORIGINAL SOURCE:

ORGANISM:Pyrococcus furiosus

SEQUENCE DESCRIPTION:SEQ ID NO:1:

GCAAAATACT TGGAGCTTGA AGAGGGAGGA GTTATAATGC AAGCATTCTA TTGGGATGTT 60

CCAGGGGGAG GAATTTGGTG GGATCATATA AGATCGAAGA TTCCTGAATG GTATGAAGCT 120

GGAATCTCTG CAATATGGCT ACCTCCACCA AGCAAGGGGA TGAGTGGAGG ATATTCAATG 180

GGCTACGATC CCTATGATTA CTTTGATCTC GGCGAGTACT ACCAGAAGGG AACTGTAGAG 240

ACGCGTTTTG GATCAAAAGA AGAACTAGTG AGATTGATAC AAACTGCCCA TGCCTATGGA 300

ATAAAGGTAA TCGCCGATGT AGTTATAAAC CACAGGGCTG GTGGTGACCT AGAATGGAAC 360

CCCTTCGTTG GAGATTACAC ATGGACAGAC TTTTCTAAAG TTGCCTCAGG GAAATATACA 420

GCTAACTATC TGGACTTCCA TCCAAACGAG CTTCATTGTT GTGACGAAGG AACCTTTGGA 480

GGATTTCCAG ATATATGTCA TCACAAAGAG TGGGATCAGT ACTGGCTATG GAAGAGCAAT 540

GAGAGTTATG CTGCTTATTT AAGAAGCATA GGATTTGATG GTTGGAGATT TGACTATGTT 600

AAGGGCTATG GAGCTTGGGT TGTCAGAGAC TGGCTTAATT GGTGGGGAGG TTGGGCAGTT 660

GGAGAGTACT GGGACACAAA TGTAGATGCA CTACTAAGCT GGGCATATGA GAGTGGTGCA 720

AAGGTCTTTG ACTTCCCGCT CTACTATAAA ATGGATGAAG CATTTGACAA TAACAACATT 780

CCAGCATTAG TCTATGCCCT ACAAAACGGA CAAACTGTAG TTTCGAGAGA TCCATTTAAG 840

GCAGTAACTT TCGTTGCCAA TCATGACACA GATATAATAT GGAACAAGTA TCCAGCATAT 900

GCGTTCATAT TGACATATGA GGGACAGCCA GTAATATTCT ACAGGGACTT TGAGGAATGG 960

CTGAACAAGG ATAAGCTAAT TAACCTCATT TGGATCCATG ATCATTTGGC AGGAGGAAGC 1020
ACAACAATTG TCTACTACGA CAACGATGAG CTCATATTTG TGAGAAATGG AGATTCTAGA 1080
AGGCCTGGGC TTATAACTTA CATTAACTTG AGCCCTAACT GGGTTGGTAG GTGGGTATAC 1140
GTTCCAAAGT TTGCAGGGGC TTGTATTCAT GAATACACTG GAAACCTAGG AGGATGGGTA 1200
GATAAAAGAG TAGATAGTAG CGGATGGGTA TACCTAGAGG CACCACCTCA CGATCCAGCT 1260
AACGGCTACT ATGGGTACTC CGTATGGAGT TATTGTGGTG TTGGG 1305

SEQ ID NO:2
SEQUENCE LENGTH: 1670
SEQUENCE TYPE:nucleic acid
STRANDEDNESS:double
TOPOLOGY:linear
MOLECULE TYPE:Genomic DNA
HYPOTHETICAL:NO
ANTI-SENSE:NO
ORIGINAL SOURCE:
ORGANISM:Pyrococcus furiosus
SEQUENCE DESCRIPTION:SEQ ID NO:2:
CCATGGCAAA ATACTTGGAG CTTGAAGAGG GAGGAGTTAT AATGCAAGCA TTCTATTGGG 60
ATGTTCCAGG GGGAGGAATT TGGTGGGATC ATATAAGATC GAAGATTCCT GAATGGTATG 120
AAGCTGGAAT CTCTGCAATA TGGCTACCTC CACCAAGCAA GGGGATGAGT GGAGGATATT 180
CAATGGGCTA CGATCCCTAT GATTACTTTG ATCTCGGCGA GTACTACCAG AAGGGAACTG 240
TAGAGACGCG TTTTGGATCA AAAGAAGAAC TAGTGAGATT GATACAAACT GCCCATGCCT 300
ATGGAATAAA GGTAATCGCC GATGTAGTTA TAAACCACAG GGCTGGTGGT GACCTAGAAT 360
GGAACCCCTT CGTTGGAGAT TACACATGGA CAGACTTTTC TAAAGTTGCC TCAGGGAAAT 420
ATACAGCTAA CTATCTGGAC TTCCATCCAA ACGAGCTTCA TTGTTGTGAC GAAGGAACCT 480
TTGGAGGATT TCCAGATATA TGTCATCACA AAGAGTGGGA TCAGTACTGG CTATGGAAGA 540
GCAATGAGAG TTATGCTGCT TATTTAAGAA GCATAGGATT TGATGGTTGG AGATTTGACT 600
ATGTTAAGGG CTATGGAGCT TGGGTTGTCA GAGACTGGCT TAATTGGTGG GGAGGTTGGG 660

CAGTTGGAGA GTACTGGGAC ACAAATGTAG ATGCACTACT AAGCTGGGCA TATGAGAGTG 720
GTGCAAAGGT CTTTGACTTC CCGCTCTACT ATAAAATGGA TGAAGCATTT GACAATAACA 780
ACATTCCAGC ATTAGTCTAT GCCCTACAAA ACGGACAAAC TGTAGTTTCG AGAGATCCAT 840
TTAAGGCAGT AACTTTCGTT GCCAATCATG ACACAGATAT AATATGGAAC AAGTATCCAG 900
CATATGCGTT CATATTGACA TATGAGGGAC AGCCAGTAAT ATTCTACAGG GACTTTGAGG 960
AATGGCTGAA CAAGGATAAG CTAATTAACC TCATTTGGAT CCATGATCAT TTGGCAGGAG 1020
GAAGCACAAC AATTGTCTAC TACGACAACG ATGAGCTCAT ATTTGTGAGA AATGGAGATT 1080
CTAGAAGGCC TGGGCTTATA ACTTACATTA ACTTGAGCCC TAACTGGGTT GGTAGGTGGG 1140
TATACGTTCC AAAGTTTGCA GGGGCTTGTA TTCATGAATA CACTGGAAAC CTAGGAGGAT 1200
GGGTAGATAA AAGAGTAGAT AGTAGCGGAT GGGTATACCT AGAGGCACCA CCTCACGATC 1260
CAGCTAACGG CTACTATGGG TACTCCGTAT GGAGTTATTG TGGTGTTGGG TGACTTTTTC 1320
TTTTTTCTTT TTAACAATGG GAGAAGTGCA AATACTGCGA CAATTCCTGG GCCGCATACA 1380
GGAGTTTCTG GAGCGGCTTC ATTCAATATT ACTGTTTTAT TTCCAATTCC ATACAACGTG 1440
ACATTTGTTG GTTGGAGAGG TTCCCAGGGA AGTCCCTTAT ATAGTTGAAC TGTAAAAGTT 1500
TTGTTTCTGG GGAGGGGAAC CACGGCAAAG TAGTGTCCCG TTGGAGATTT CAAGAGGGGT 1560
CTCGGCTCTG AGAGTTCTGG TATGTTCCCC TTGACCCAAA GGTAACCAGT GTAGTTTCCT 1620
TGTAGGACTA TTAGTGCTGG GATATCATTG CTAGAATAGT ACCTAAGCTT 1670

SEQ ID NO:3
SEQUENCE LENGTH: 2134
SEQUENCE TYPE:nucleic acid
STRANDEDNESS:double
TOPOLOGY:linear
MOLECULE TYPE:Genomic DNA
HYPOTHETICAL:NO
ANTI-SENSE:NO
ORIGINAL SOURCE:
ORGANISM:Pyrococcus furiosus
SEQUENCE DESCRIPTION:SEQ ID NO:3:

CGGCCACCAT TGTAACGTCA TCGTGAGTTG GCCCGAGGCC TCCCGAAATA ATAAGAACAT 60
CGGGTTTTCT GTTAAGGGAT TCTAAAATTA CAGACCTTAT ATCCTCCACG TCATCGCCAA 120
CAGTAGTTAT TCTCTTAACG AGGTATCCTC TCTCCGTTAA TTTCTTCGCT ATGTGTGCTG 180
AGTTGCTGTT TACAGTATTC CCTGTTAAAA GTTCATCACC TACGGTAATT ATTTCCGCGA 240
ACATTGGTTC TCCCCAGGAA TTGTTTTTAT CAAGAGTTTA TTAGATTTTG ACGTGCGTTG 300
ATGAACATTT ATGTTCACAT GATCATAACA GAAAAATTTA TATGTATCAT CACCAGTGAT 360
ACATTATGAG ACTTTGGTGT ATGGAGGTGA TCACGTGAAC ATAAAGAAAT TAACACCCCT 420
CCTAACTCTA TTACTGTTTT TTATAGTACT AGCAAGTCCA GTAAGTGCAG CAAAATACTT 480
GGAGCTTGAA GAGGGAGGAG TTATAATGCA AGCATTCTAT TGGGATGTTC CAGGGGGAGG 540
AATTTGGTGG GATCATATAA GATCGAAGAT TCCTGAATGG TATGAAGCTG GAATCTCTGC 600
AATATGGCTA CCTCCACCAA GCAAGGGGAT GAGTGGAGGA TATTCAATGG GCTACGATCC 660
CTATGATTAC TTTGATCTCG GCGAGTACTA CCAGAAGGGA ACTGTAGAGA CGCGTTTTGG 720
ATCAAAAGAA GAACTAGTGA GATTGATACA AACTGCCCAT GCCTATGGAA TAAAGGTAAT 780
CGCCGATGTA GTTATAAACC ACAGGGCTGG TGGTGACCTA GAATGGAACC CCTTCGTTGG 840
AGATTACACA TGGACAGACT TTTCTAAAGT TGCCTCAGGG AAATATACAG CTAACTATCT 900
GGACTTCCAT CCAAACGAGC TTCATTGTTG TGACGAAGGA ACCTTTGGAG GATTTCCAGA 960
TATATGTCAT CACAAAGAGT GGGATCAGTA CTGGCTATGG AAGAGCAATG AGAGTTATGC 1020
TGCTTATTTA AGAAGCATAG GATTTGATGG TTGGAGATTT GACTATGTTA AGGGCTATGG 1080
AGCTTGGGTT GTCAGAGACT GGCTTAATTG GTGGGGAGGT TGGGCAGTTG GAGAGTACTG 1140
GGACACAAAT GTAGATGCAC TACTAAGCTG GGCATATGAG AGTGGTGCAA AGGTCTTTGA 1200
CTTCCCGCTC TACTATAAAA TGGATGAAGC ATTTGACAAT AACAACATTC CAGCATTAGT 1260
CTATGCCCTA CAAAACGGAC AAACTGTAGT TTCGAGAGAT CCATTTAAGG CAGTAACTTT 1320
CGTTGCCAAT CATGACACAG ATATAATATG GAACAAGTAT CCAGCATATG CGTTCATATT 1380
GACATATGAG GGACAGCCAG TAATATTCTA CAGGGACTTT GAGGAATGGC TGAACAAGGA 1440
TAAGCTAATT AACCTCATTT GGATCCATGA TCATTTGGCA GGAGGAAGCA CAACAATTGT 1500
CTACTACGAC AACGATGAGC TCATATTTGT GAGAAATGGA GATTCTAGAA GGCCTGGGCT 1560
TATAACTTAC ATTAACTTGA GCCCTAACTG GGTTGGTAGG TGGGTATACG TTCCAAAGTT 1620
TGCAGGGGCT TGTATTCATG AATACACTGG AAACCTAGGA GGATGGGTAG ATAAAAGAGT 1680
AGATAGTAGC GGATGGGTAT ACCTAGAGGC ACCACCTCAC GATCCAGCTA ACGGCTACTA 1740

TGGGTACTCC GTATGGAGTT ATTGTGGTGT TGGGTGACTT TTTCTTTTTT CTTTTTAACA 1800
ATGGGAGAAG TGCAAATACT GCGACAATTC CTGGGCCGCA TACAGGAGTT TCTGGAGCGG 1860
CTTCATTCAA TATTACTGTT TTATTTCCAA TTCCATACAA CGTGACATTT GTTGGTTGGA 1920
GAGGTTCCCA GGGAAGTCCC TTATATAGTT GAACTGTAAA AGTTTTGTTT CTGGGGAGGG 1980
GAACCACGGC AAAGTAGTGT CCCGTTGGAG ATTTCAAGAG GGGTCTCGGC TCTGAGAGTT 2040
CTGGTATGTT CCCCTTGACC CAAAGGTAAC CAGTGTAGTT TCCTTGTAGG ACTATTAGTG 2100
CTGGGATATC ATTGCTAGAA TAGTACCTAA GCTT 2134

SEQ ID NO:4
SEQUENCE LENGTH: 460
SEQUENCE TYPE:amino acid
STRANDEDNESS:single
TOPOLOGY:linear
MOLECULE TYPE:peptide
ORIGINAL SOURCE:
ORGANISM:Pyrococcus furiosus
SEQUENCE DESCRIPTION:SEQ ID NO:4:
Met Asn Ile Lys Lys Leu Thr Pro Leu Leu Thr Leu Leu Leu Phe
1               5                   10                  15
Phe Ile Val Leu Ala Ser Pro Val Ser Ala Ala Lys Tyr Leu Glu
                20                  25                  30
Leu Glu Glu Gly Gly Val Ile Met Gln Ala Phe Tyr Trp Asp Val
                35                  40                  45
Pro Gly Gly Gly Ile Trp Trp Asp His Ile Arg Ser Lys Ile Pro
                50                  55                  60
Glu Trp Tyr Glu Ala Gly Ile Ser Ala Ile Trp Leu Pro Pro Pro
                65                  70                  75
Ser Lys Gly Met Ser Gly Gly Tyr Ser Met Gly Tyr Asp Pro Tyr
                80                  85                  90

Asp Tyr Phe Asp Leu Gly Glu Tyr Tyr Gln Lys Gly Thr Val Glu
                95                  100                 105
Thr Arg Phe Gly Ser Lys Glu Glu Leu Val Arg Leu Ile Gln Thr
                110                 115                 120
Ala His Ala Tyr Gly Ile Lys Val Ile Ala Asp Val Val Ile Asn
                125                 130                 135
His Arg Ala Gly Gly Asp Leu Glu Trp Asn Pro Phe Val Gly Asp
                140                 145                 150
Tyr Thr Trp Thr Asp Phe Ser Lys Val Ala Ser Gly Lys Tyr Thr
                155                 160                 165
Ala Asn Tyr Leu Asp Phe His Pro Asn Glu Leu His Cys Cys Asp
                170                 175                 180
Glu Gly Thr Phe Gly Gly Phe Pro Asp Ile Cys His His Lys Glu
                185                 190                 195
Trp Asp Gln Tyr Trp Leu Trp Lys Ser Asn Glu Ser Tyr Ala Ala
                200                 205                 210
Tyr Leu Arg Ser Ile Gly Phe Asp Gly Trp Arg Phe Asp Tyr Val
                215                 220                 225
Lys Gly Tyr Gly Ala Trp Val Val Arg Asp Trp Leu Asn Trp Trp
                230                 235                 240
Gly Gly Trp Ala Val Gly Glu Tyr Trp Asp Thr Asn Val Asp Ala
                245                 250                 255
Leu Leu Ser Trp Ala Tyr Glu Ser Gly Ala Lys Val Phe Asp Phe
                260                 265                 270
Pro Leu Tyr Tyr Lys Met Asp Glu Ala Phe Asp Asn Asn Asn Ile
                275                 280                 285
Pro Ala Leu Val Tyr Ala Leu Gln Asn Gly Gln Thr Val Val Ser
                290                 295                 300
Arg Asp Pro Phe Lys Ala Val Thr Phe Val Ala Asn His Asp Thr

                305                 310                 315
Asp Ile Ile Trp Asn Lys Tyr Pro Ala Tyr Ala Phe Ile Leu Thr
                320                 325                 330
Tyr Glu Gly Gln Pro Val Ile Phe Tyr Arg Asp Phe Glu Glu Trp
                335                 340                 345
Leu Asn Lys Asp Lys Leu Ile Asn Leu Ile Trp Ile His Asp His
                350                 355                 360
Leu Ala Gly Gly Ser Thr Thr Ile Val Tyr Tyr Asp Asn Asp Glu
                365                 370                 375
Leu Ile Phe Val Arg Asn Gly Asp Ser Arg Arg Pro Gly Leu Ile
                380                 385                 390
Thr Tyr Ile Asn Leu Ser Pro Asn Trp Val Gly Arg Trp Val Tyr
                395                 400                 405
Val Pro Lys Phe Ala Gly Ala Cys Ile His Glu Tyr Thr Gly Asn
                410                 415                 420
Leu Gly Gly Trp Val Asp Lys Arg Val Asp Ser Ser Gly Trp Val
                425                 430                 435
Tyr Leu Glu Ala Pro Pro His Asp Pro Ala Asn Gly Tyr Tyr Gly
                440                 445                 450
Tyr Ser Val Trp Ser Tyr Cys Gly Val Gly
                455                 460

SEQ ID NO:5
SEQUENCE LENGTH: 25
SEQUENCE TYPE:nucleic acid
STRANDEDNESS:single
TOPOLOGY:linear
MOLECULE TYPE:Other nucleic acid(synthetic DNA)
SEQUENCE DESCRIPTION:SEQ ID NO:5:

CAGTAAGTGC CATGGCAAAA TACTT   25

SEQ ID NO:6
SEQUENCE LENGTH: 25
SEQUENCE TYPE:nucleic acid
STRANDEDNESS:single
TOPOLOGY:linear
MOLECULE TYPE:Other nucleic acid(synthetic DNA)
SEQUENCE DESCRIPTION:SEQ ID NO:6:
TTGTATCAAT CTCACTAGTT CTTCT   25

SEQ ID NO:7
SEQUENCE LENGTH: 435
SEQUENCE TYPE:amino acid
STRANDEDNESS:single
TOPOLOGY:linear
MOLECULE TYPE:peptide
ORIGINAL SOURCE:
  ORGANISM:Pyrococcus furiosus
SEQUENCE DESCRIPTION:SEQ ID NO:7:
Ala Lys Tyr Leu Glu Leu Glu Glu Gly Gly Val Ile Met Gln Ala
1               5                   10                  15
Phe Tyr Trp Asp Val Pro Gly Gly Gly Ile Trp Trp Asp His Ile
                20                  25                  30
Arg Ser Lys Ile Pro Glu Trp Tyr Glu Ala Gly Ile Ser Ala Ile
                35                  40                  45
Trp Leu Pro Pro Pro Ser Lys Gly Met Ser Gly Gly Tyr Ser Met
                50                  55                  60
Gly Tyr Asp Pro Tyr Asp Tyr Phe Asp Leu Gly Glu Tyr Tyr Gln

                65                  70                  75
Lys Gly Thr Val Glu Thr Arg Phe Gly Ser Lys Glu Glu Leu Val
                80                  85                  90
Arg Leu Ile Gln Thr Ala His Ala Tyr Gly Ile Lys Val Ile Ala
                95                  100                 105
Asp Val Val Ile Asn His Arg Ala Gly Gly Asp Leu Glu Trp Asn
                110                 115                 120
Pro Phe Val Gly Asp Tyr Thr Trp Thr Asp Phe Ser Lys Val Ala
                125                 130                 135
Ser Gly Lys Tyr Thr Ala Asn Tyr Leu Asp Phe His Pro Asn Glu
                140                 145                 150
Leu His Cys Cys Asp Glu Gly Thr Phe Gly Gly Phe Pro Asp Ile
                155                 160                 165
Cys His His Lys Glu Trp Asp Gln Tyr Trp Leu Trp Lys Ser Asn
                170                 175                 180
Glu Ser Tyr Ala Ala Tyr Leu Arg Ser Ile Gly Phe Asp Gly Trp
                185                 190                 195
Arg Phe Asp Tyr Val Lys Gly Tyr Gly Ala Trp Val Val Arg Asp
                200                 205                 210
Trp Leu Asn Trp Trp Gly Gly Trp Ala Val Gly Glu Tyr Trp Asp
                215                 220                 225
Thr Asn Val Asp Ala Leu Leu Ser Trp Ala Tyr Glu Ser Gly Ala
                230                 235                 240
Lys Val Phe Asp Phe Pro Leu Tyr Tyr Lys Met Asp Glu Ala Phe
                245                 250                 255
Asp Asn Asn Asn Ile Pro Ala Leu Val Tyr Ala Leu Gln Asn Gly
                260                 265                 270
Gln Thr Val Val Ser Arg Asp Pro Phe Lys Ala Val Thr Phe Val
                275                 280                 285

Ala Asn His Asp Thr Asp Ile Ile Trp Asn Lys Tyr Pro Ala Tyr
                290                 295                 300
Ala Phe Ile Leu Thr Tyr Glu Gly Gln Pro Val Ile Phe Tyr Arg
                305                 310                 315
Asp Phe Glu Glu Trp Leu Asn Lys Asp Lys Leu Ile Asn Leu Ile
                320                 325                 330
Trp Ile His Asp His Leu Ala Gly Gly Ser Thr Thr Ile Val Tyr
                335                 340                 345
Tyr Asp Asn Asp Glu Leu Ile Phe Val Arg Asn Gly Asp Ser Arg
                350                 355                 360
Arg Pro Gly Leu Ile Thr Tyr Ile Asn Leu Ser Pro Asn Trp Val
                365                 370                 375
Gly Arg Trp Val Tyr Val Pro Lys Phe Ala Gly Ala Cys Ile His
                380                 385                 390
Glu Tyr Thr Gly Asn Leu Gly Gly Trp Val Asp Lys Arg Val Asp
                395                 400                 405
Ser Ser Gly Trp Val Tyr Leu Glu Ala Pro Pro His Asp Pro Ala
                410                 415                 420
Asn Gly Tyr Tyr Gly Tyr Ser Val Trp Ser Tyr Cys Gly Val Gly
                425                 430                 435

## Claims

1. An isolated DNA selected from the group consisting of:
(a) a DNA encoding a hyperthermostable α-amylase and having a nucleotide sequence selected from the group consisting of the sequences represented by SEQ ID No: 1, SEQ ID No: 2 and SEQ ID No: 3 of the sequence listing;
(b) a DNA capable of hybridizing to the DNA of (a) under stringent conditions and which encodes hyperthermostable α-amylase.
